# EUROPÄISCHE PATENTSCHRIFT

(11) **EP 3 052 159 B1**
(45) Veröffentlichungstag und Bekanntmachung des Hinweises auf die Patenterteilung: **13.12.2017**
(21) Anmeldenummer: 14792385.8
(22) Anmeldetag: 02.10.2014
(51) Int. Cl.: A61M 1/36

(54) **MEDIZINISCHES GERÄT UND VERFAHREN ZUR DETEKTION DES FÜLLSTANDES EINER BLASENKAMMER**
MEDICAL DEVICE AND METHOD FOR DETECTING THE FILL LEVEL OF A BUBBLE CHAMBER
APPAREIL MÉDICAL ET PROCÉDÉ DE DÉTECTION DU NIVEAU DE REMPLISSAGE D'UNE CHAMBRE À BULLES

(30) Priorität: 04.10.2013 DE 102013016469
(43) Veröffentlichungstag der Anmeldung: 10.08.2016
(73) Patentinhaber: Fresenius Medical Care Deutschland GmbH, 61352 Bad Homburg (DE)
(72) Erfinder: HEIDE, Alexander, 65817 Eppstein (DE)
(74) Vertreter: Behr, Wolfgang
(86) Internationale Anmeldenummer: PCT/EP2014/002687
(87) Internationale Veröffentlichungsnummer: WO 2015/049056

(56) Entgegenhaltungen:
- WO-A1-2007/133259
- WO-A1-2009/052496
- DE-A1-102007 039 581
- US-A1- 2011 257 579

## Beschreibung

Die vorliegende Erfindung betrifft ein medizinisches Gerät, insbesondere eine Blutbehandlungsvorrichtung für die extrakorporale Blutbehandlung. An das medizinische Gerät ist ein medizinischer Schlauchsatz ankoppelbar, durch welchen das medizinische Gerät mindestens eine medizinische Flüssigkeit transportiert.
Solche Schlauchsätze weisen oftmals eine Blasenkammer -auf, die dazu dient, eventuell in dem durch den Schlauchsatz gebildeten Fluidkreislauf auftretende Luftblasen aus der transportierten Flüssigkeit abzuscheiden.
Entsprechende Blutbehandlungsvorrichtungen weisen unter anderem eine venöse Blasenkammer auf, die dazu dient, eventuell im extrakorporalen Blutkreislauf auftretende Luftblasen aus dem Blut abzuscheiden und somit Luftembolien bei der Rückleitung des Bluts in den Patienten zu verhindern. Der Flüssigkeitsspiegel des Bluts in der Blasenkammer soll einerseits einen bestimmten Minimalwert nicht unterschreiten, damit die Blasenkammer nicht leer läuft und andererseits einen bestimmten Maximalwert nicht überschreiten, damit die Blasenkammer nicht vollständig mit Flüssigkeit befüllt ist, sondern stets ein Flüssigkeitsspiegel vorliegt. Daher muss der Füllstand ständig überwacht werden. Der Pegel in einer Blasenkammer wird in der Regel bei Bedarf durch Zugeben oder Ablassen von Luft in den Bereich oberhalb des Löslichkeitsspiegels justiert. Beispielsweise befinden sich zur Überwachung des Füllstandes der venösen Blasenkammer Ultraschallsensoren an der maschinenseitigen Aufnahme für die venöse Blasenkammer. Aus der Änderung des Ultraschallsignals wird auf den Füllstand der venösen Kammer geschlossen. Diese maschinenseitige Ultraschallmesstechnik ist jedoch aufwendig und muss verlässlich an die venöse Blasenkammer angekoppelt werden. Insbesondere bedingt die Ultraschallmessung ein korrektes Einlegen der Blasenkammer in die maschinenseitige Aufnahme, so dass die Blasenkammer eine definierte Position gegenüber den Ultraschallsensoren einnimmt. Dies erfordert einerseits entsprechende konstruktive Maßnahmen zur räumlichen Festlegung der Blasenkammer an der Blutbehandlungsvorrichtung und andererseits ein aufmerksames Aufrüsten des Blutschlauchsatzes an der Blutbehandlungsvorrichtung. Dennoch bleibt dieser Arbeitsgang fehleranfällig, so dass es zu Fehlmessungen des Füllstandes kommen kann.

Aus der DE 37 20 667 A1 ist es bekannt, an Stelle einer Detektion des Pegels in einer Blasenkammer das Vorhandensein von Luftblasen im Blutstrom, welcher durch den Blutschlauchsatz fließt, durch eine vor der Blasenkammer angeordnete, an den Blutschlauchsatz angekoppelte Ultraschall-Meßstrecke zu detektieren. Die Signale der Ultraschall-Meßstrecke werden dann aufsummiert und mit Grenzwerten verglichen, um ggf. einen Alarm auszulösen.

Aus der US 2003/0009123 A1 ist der Einsatz einer Ultraschall-Meßstrecke zur Detektion von Luftblasen im Blutstrom bekannt. In der US 2008/0195021 A1 werden Ultraschallemitter und Sender zu Detektion einer Nadeldiskonnektion eingesetzt.

Aus der US 2011/0257579 A1 ist es bekannt, den Füllstand einer Blasenkammer durch einen Ultraschall-Emitter und einen Ultraschall-Sensor zu bestimmen, welche an der Wand der Blasenkammer angeordnet werden, wobei der Füllstand durch seinen Einfluss auf die Phasenlage des in der Wand übertragenen Signals bestimmt wird.

Aus der WO 2007/133259 A1 ist es bekannt, den Füllstand einer Kammer über mehrere in der Wand der Kammer angeordnete Sensoren zu bestimmen, welche jeweils einen Ultraschall-Emitter und einen Ultraschall-Sensor aufweisen. Dabei wird über das Reflexionsverhalten ermittelt, ob der Füllstand bis zur Höhe des jeweiligen Sensors reicht oder nicht.
Aus der WO 2009/052496 A1 ist es bekannt, den Füllstand eines Beutels oder einer Kammer über das Reflexionsverhalten mittels eines Sensors zu bestimmen, welcher einen Ultraschall-Emitter und einen Ultraschall-Sensor aufweist.
Aufgabe der vorliegenden Erfindung ist es nun, die Detektion des Pegels in einer Blasenkammer, beispielsweise einer venösen Blasenkammer eines extrakorporalen Blutkreislaufs, die Teil des Disposable-Blutschlauchsatzes für die Durchführung einer extrakorporalen Blutbehandlung ist, derart weiterzubilden, dass dies einfach und funktionssicher durchführbar ist.
Erfindungsgemäß wird diese Aufgabe durch ein medizinisches Gerät mit den Merkmalen des Anspruchs 1 gelöst. Jegliche Beschreibung von Merkmalen als erfindungsgemäß und/oder vorteilhaft ist im Folgenden so zu verstehen, dass Schutz begehrt wird für die in den Ansprüchen definierten Gegenstände. Demnach wird ein medizinisches Gerät mit einer Steuer- und Recheneinheit bereitgestellt, die mindestens einen Körperschallemitter und mindestens einen Körperschallsensor aufweist, wobei diese jeweils so konfiguriert sind, dass sie an Ankoppelstellen eines an das medizinische Gerät ankoppelbaren medizinischen Schlauchsatzes ankoppelbar sind, wobei über die Steuer- und Recheneinheit der Füllstand einer im Schlauchsatz angeordneten Blasenkammer auf der Grundlage der Messung des Körperschalls am Schlauchsatz bestimmbar ist, indem über den Körperschallemitter ein Körperschall über eine Ankoppelstelle in den Schlauchsatz vor der Blasenkammer eingekoppelt wird und der sich im Fluidkreislauf ausbreitende Körperschall über den an eine in Körperschallausbreitungsrichtung nach der Blasenkammer angeordnete Ankoppelstelle gekoppelten Körperschallsensor aufgenommen und der Steuer- und Recheneinheit zur Bestimmung der Füllhöhe zugeleitet wird. Diese erfindungsgemäße Lösung beruht auf der Auswertung von Körperschall, der sich im Schlauchsatz fortpflanzt. Solche Auswertungen der Ausbreitung von Körperschall im Schlauchsatz können beispielsweise zur Überwachung der Integrität des Schlauchsatzes eingesetzt werden. Der Körperschall kann beispielsweise mittels einer Schallwelle an dem medizinischen Gerät in den Blutschlauchsatz eingekoppelt werden.
Untersuchungen haben überraschend gezeigt, dass sich der Körperschall nicht nur über die Flüssigkeit im Schlauchsatz fortpflanzt, sondern maßgeblich auch über längere Strecken in den Wandungen des Schlauches und in den Wandungen der Komponenten des Schlauchsatz weitergeleitet wird, wie beispielsweise in den Wandungen der Blasenkammer und in den Wandungen des Dialysators. Dabei wirken die Wandungen der Bauteile jedoch im Vergleich zur Schallleitung der Flüssigkeit im Schlauchsatz eher dämpfend auf die Schallausbreitung. Diese Eigenschaften nutzt die Erfindung aus. Erfindungsgemäss lässt sich der Füllstand einer im Schlauchsatz angeordneten Blasenkammer bestimmen. Dieser Aspekt der Erfindung berücksichtigt, dass sich das Dämpfungsverhalten der Blasenkammer signifikant und reproduzierbar mit abnehmendem Füllstand verstärkt. Je höher der Füllstand ist, desto geringer ist die Dämpfung. Die vorliegende Erfindung beruht daher darauf, dass vom gemessenen Dämpfungsverhalten der Blasenkammer auf deren Füllstand geschlossen wird. Das Dämpfungsverhalten wird ermittelt, indem die Amplitude des Körperschalls in einer Schallausbreitungsrichtung hinter der zu vermessenden Blasenkammer gemessen wird, wobei der gemessene Körperschall beim Durchlaufen der Blasenkammer in Abhängigkeit vom Füllstand unterschiedlich stark gedämpft wird.

Erfindungsgemäß wird dabei der über den Körperschallemitter in den Schlauchsatz eingekoppelte und sich im Fluidkreislauf ausbreitende Körperschall über den Körperschallsensor aufgenommen und der Steuer- und Recheneinheit zur Bestimmung der Füllhöhe zugeleitet. Insbesondere wird die Füllhöhe der Blasenkammer daher durch Auswertung eines Signals des Körperschallsensors bestimmt. Gemäß einer möglichen Ausführungsform der vorliegenden Erfindung bestimmt die Steuer-und Recheneinheit die Füllhöhe der Blasenkammer durch Vergleich mit mindestens einer abgespeicherten Kennlinie. Eine solche Kennlinie, welche die Füllhöhe in Abhängigkeit von der gemessenen Amplitude des Signals des Körperschallsensors wiedergeben kann, kann in einem Speicher des medizinischen Gerätes hinterlegt sein. Dabei können in dem medizinischen Gerät mehrere Kennlinien implementiert sein, welche für unterschiedliche, mit dem medizinischen Gerät einsetzbare Schlauchsätze eingesetzt werden. Bevorzugt umfasst das medizinische Gerät dabei eine Erfassungseinrichtung zur Erfassung des angekoppelten Schlauchsatzes, insbesondere eine Eingabeeinheit oder einen Sensor, wobei die Kennlinie in Abhängigkeit von dem erfassten Schlauchsatz ausgewählt wird.

Gemäß einer ersten Ausführungsform der vorliegenden Erfindung handelt es sich bei dem Körperschallemitter um eine okkludierende Pumpe, insbesondere eine Rollenpumpe, welche an einen Pumpabschnitt des Schlauchsatzes ankoppelbar ist. Solche okkludierenden Pumpen erzeugten Drucksignale, welche sich als Körperschall im Schlauchsatz ausbreiten.

Gemäß einer zweiten Ausführungsform der vorliegenden Erfindung handelt es sich bei dem Körperschallemitter um einen elektronisch angesteuerten Schallerzeuger. Insbesondere wird dabei durch die Steuer- und Recheneinheit ein Ansteuersignal erzeugt, welches der Schallerzeuger in Körperschall umwandelt. Bevorzugt umfasst der Schallerzeuger ein Piezoelement. Der Schallerzeuger kann dabei stromaufwärts oder stromabwärts einer Pumpe am Schlauchsatz ankoppelbar sein. Bei der Pumpe kann es sich in diesem Fall auch um eine nicht-okkludierende Pumpe handeln, insbesondere eine Impellerpumpe.

Insbesondere kann dabei ein separates Körperschallsignal mit definierter Frequenz eingespeist werden. Als besonders geeignet für die Einspeisung in den Schlauchsatz hat sich eine sinusförmige Frequenz im Frequenzbereich von mindestens 80 Hz, insbesondere 160 Hz erwiesen. Auch ein rechteckiges Pulssignal ist geeignet.

Als Körperschallsensor ist beispielsweise ein Piezofoliensensor geeignet, der an den Schlauchsatz ankoppelbar ist.

In einer weiteren Ausführungsform der vorliegenden Erfindung können auch zwei Körperschallemitter vorgesehen sein. Bevorzugt erzeugen diese unterschiedliche Signale, so dass die von den beiden Körperschallemittern ausgesandten Signale im gemessenen Signal unterscheidbar bleiben. Bspw. kann dabei eine okkludierende Pumpe als erster Körperschallemitter und ein elektronisch angesteuerter Schallerzeuger als zweiter Körperschallemitter eingesetzt werden.

Gemäß einem weiteren Aspekt der Erfindung kann als Zustand des Abschnitts des Schlauchsatzes insgesamt die Integrität des Schlauchsatzes und/oder des mittels des Schlauchsatzes gebildeten Fluidkreislaufs bestimmt werden.

Vorteilhaft kann der mindestens eine Emitter und der mindestens eine Sensor in das medizinische Gerät derart integriert sein, dass diese beim Einlegen eines Schlauchsatzes jeweils an vorgesehenen Ankoppelstellen an den Schlauchsatz ankoppelbar sind.

In der Blasenkammer kann der Füllstand jeglicher Flüssigkeit, insbesondere von Blut, physiologischer Kochsalzlösung, Primingflüssigkeit, Substitutionsflüssigkeit oder Medikamentenlösung bestimmbar sein. Die vorliegenden Erfindung kann daher in beliebigen medizinische Geräten, bei welchen ein Schlauchsatz mit einer Blasenkammer eingesetzt wird, zur Anwendung kommen.

In einer bevorzugten Ausführungsform handelt es sich bei dem medizinischen Gerät um eine Blutbehandlungsvorrichtung. Insbesondere kann es sich dabei um ein Dialysegerät handeln. Bei dem Schlauchsatz kann es sich dabei bspw. um einen Blutschlauchsatz, einen Dialysatschlauchsatz, einen Substituatschlauchsatz und/oder einen Filtratschlauchsatz handeln.

Besonders bevorzugt handelt es sich bei dem medizinischen Gerät um eine Blutbehandlungsvorrichtung für die extrakorporale Blutbehandlung und bei dem Schlauchsatz um einen extrakorporalen Blutschlauchsatz.

Gemäß einem Aspekt der vorliegenden Erfindung kann die Steuer-und Recheneinheit dabei zusätzlich zu der Füllstandsbestimmung den Zustand eines Patientenzugangs überwachen. Insbesondere erfolgt auch die Überwachung des Patientenzugangs auf der Grundlage der Messung des Körperschalls am Schlauchsatz, und insbesondere durch eine Auswertung des Signals des Körperschallsensors.

Insbesondere detektiert die Steuer- und Recheneinheit dabei eine Nadeldiskonnektion, wenn sich die Schallausbreitung im extrakorporalen Blutkreislauf aufgrund des fehlerhaften Patientenzugangs ändert und/oder die gemessene Amplitude des Körperschalls abnimmt oder ausbleibt. Insbesondere kann dabei eine venöse Nadeldiskonnektion detektiert werden.

An einen extrakorporalen Blutkreislauf kann im Falle einer venösen Nadeldiskonnektion eine Verringerung der gemessenen Amplitude des Körperschalls um rund 30 % erwartet werden, so dass in diesem Fehlerfall ein signifikanter Hinweis auf eine venöse Nadeldiskonnektion vorliegt. Insbesondere schließt die Steuer- und Recheneinheit daher auf eine Nadeldiskonnektion, wenn eine Verringerung der gemessenen Amplitude des Körperschalls um einen Schwellwert, welcher zwischen 20 % und 60 % der gemessenen Amplitude und/oder eines Basissignal liegt, erkannt wird.

In einer bevorzugten Ausführungsform ist vorgesehen, dass die Steuer- und Recheneinheit bei Detektion einer Nadeldiskonnektion die Blutpumpe stoppt und/oder einen Alarm auslöst.

Erfindungsgemäß kann das Signal des Körperschallsensors zur Überwachung des Patientenzugangs und zur gleichzeitigen der Bestimmung der Füllhöhe der Blasenkammer in unterschiedlicher Weise ausgewertet werden. Dabei werden bevorzugt unterschiedliche Signalanteile und/oder sich in ihrem zeitlichen Verlauf unterscheidende Änderungen des Signals den beiden unterschiedlichen Aspekten zugeordnet.

Dabei kann das Signal zur Detektion einer Nadeldiskonnektion auf schnellere Amplitudenänderungen hin überwacht werden, während Änderungen der Füllhöhe der Blasenkammer anhand langsamerer Amplitudenänderungen erfasst werden.

Die vorliegende Erfindung macht sich hier zunutze, dass die gemessene Amplitude zwar abhängig von den Pegelständen in der Blasenkammer sinkt oder steigt, was jedoch kontinuierlich und mit deutlich niedrigerer Frequenz und Stärke erfolgt als der Amplitudensprung, der bei einer Nadeldiskonnektion auftritt. Daher können die beiden Vorgänge durch entsprechende Auswertung des Signals des Körperschallsensors gut voneinander unterschieden werden.

Zur Detektion einer Nadeldiskonnektion kann das aktuell gemessene Signal dabei mit einem Basissignal verglichen werden. Bevorzugt wird dieses während des Behandlungsverlaufs aktualisiert und/oder auf Grundlage eines zeitlichen Mittelwertes gebildet. Das Basissignal gibt damit die Änderung des Messsignals aufgrund von Änderungen des Pegelstands in der Blasenkammer wieder.

Dabei kann das Basissignal nach einer gesteuerten Änderung des Pegels in einer Blasenkammer des extrakorporalen Blutkreislaufs aktualisierbar sein, und insbesondere nach einer Entlüftung der Blasenkammer. Bevorzugt wird die Aktualisierung durch die Steuer- und Recheneinheit automatisiert durchgeführt.

Das Basissignal, welches zur Bestimmung des Pegelstandes und/oder zum Vergleich mit dem aktuell gemessenen Signal herangezogen wird, wird dabei bevorzugt auf Grundlage eines zeitlichen Mittelwertes des gemessenen Signals gebildet.

Gemäß der vorliegenden Erfindung kann dabei ein einziger Körperschallemitter eingesetzt werden, dessen Signal sich über den extrakorporalen Blutschlauchsatz und den Patientenzugang bis zum Körperschallsensor ausbreitet.

Das Signal kann sich in zwei Wegen, zum einen über den extrakorporalen Blutschlauchsatz, und zum anderen über den Patientenzugang zum Körperschallsensor ausbreiten. Dabei kann sich der Körperschall insbesondere auf einem ersten Weg über den extrakorporalen Blutschlauchsatz durch die Blasenkammer zum Körperschallsensor ausbreiten, und zum anderen auf einem zweiten Weg über den Patientenzugang zum Körperschallsensor, ohne durch die Blasenkammer zu gehen.

Das Signal kann sich dabei aber auch in nur einem Weg über den extrakorporalen Blutschlauchsatz und über den Patientenzugang zum Körperschallsensor ausbreiten. Dabei kann der Körperschall insbesondere die Blasenkammer passieren, bevor oder nachdem er den Patientenzugang passiert.

Erfindungsgemäß können aber auch zwei Körperschallemitter mit unterschiedlichen Signalverläufen eingesetzt werden, insbesondere eine okkludierende Blutpumpe und ein stromaufwärts oder stromabwärts der Blutpumpe angeordneter weiterer Körperschallemitter.

Bevorzugt werden die jeweils auf die Körperschallemitter entfallenden Signalanteile getrennt und zur Überwachung des Patientenzugangs sowie zur gleichzeitigen der Bestimmung der Füllhöhe der Blasenkammer separat ausgewertet. Insbesondere kann sich das Signal des einen Körperschallemitters verstärkt auf einem ersten Weg über den den extrakorporalen Blutschlauchsatz durch die Blasenkammer zum Körperschallsensor ausbreiten, und das Signal des anderen Körperschallemitters auf einem zweiten Weg über den Patientenzugang zum Körperschallsensor, ohne durch die Blasenkammer zu gehen. Das Signal, welches durch den Patientenzugang geht, wird daher nicht durch den Füllstand der Blasenkammer beeinflusst.

Gemäß einem anderen Aspekt der Erfindung ist ein medizinisches Gerät mit einem Schlauchsatz, insbesondere eine Blutbehandlungsmaschine mit einem Blutschlauchsatz, wie sie oben beschrieben wurden, unter Schutz gestellt, wobei der Schlauchsatz mindestens eine Ankoppelstelle für einen Körperschallemitter und mindestens eine Ankoppelstelle für einen Körperschallsensor aufweist. Vorteilhaft ist die Ankoppelstelle für den Körperschallemitter am arteriellen Zweig des Blutschlauchsatzes stromaufwärts der Blutpumpe angeordnet, oder die Blutpumpe dient als Körperschallemitter.

Gemäß einem weiteren Aspekt der Erfindung ist die Ankoppelstelle für den Körperschallsensor stromabwärts der venösen Blasenkammer angeordnet. In der Regel wird sich der Körperschall ausgehend von dem maschinenseitigen Körperschallemitter sowohl in dem arteriellen wie auch in dem venösen Zweig des extrakorporalen Blutschlauchsatzes gleichzeitig ausbreiten, so dass auch Schallwellen auf den Körperschallsensor auftreffen, die die Blasenkammer nicht durchlaufen haben. Entscheidend für die Funktion der Erfindung ist, dass zumindest ein Teil der Körperschallwellen die Blasenkammer durchläuft und gedämpft wird, bevor sie auf den Körperschallsensor treffen.

Gemäß einem weiteren Aspekt der Erfindung ist die Ankoppelstelle für den Körperschallsensor stromaufwärts der venösen Blasenkammer angeordnet. In diesem Fall haben die Schallwellen, welche sich vom Körperschallemitter über den Patientenzugang in die venöse Leitung ausbreiten, vor dem Eintreffen bei dem Körperschallemitter die Blasenkammer durchlaufen. Das gemessene Signal umfasst damit Signalanteile, welche auf dem Durchlaufen der Blasenkammer beruhen, und Signalanteile, welche auf dem Durchlaufen des Patientenzugangs beruhen. Wie oben beschrieben können die auf eine Pegeländerung in der Blasenkammer und die auf eine Nadeldiskonnektion zurückgehenden Veränderungen jedoch voneinander unterschieden werden.

Die Anwendung der Erfindung ist natürlich nicht auf venöse Blasenkammern beschränkt, sondern kann auch an anderen Stellen des extrakorporalen Blutkreislaufs erfolgen, beispielsweise auch im arteriellen Zweig des extrakorporalen Blutkreislaufs. Es ist dann ein Körperschallsensor bei der arteriellen Blasenkammer anzuordnen, derart, dass zumindest ein Teil der Körperschallwellen die arterielle Blasenkammer durchläuft, bevor er auf den Körperschallsensor trifft.

Gemäß der Erfindung kann also auf die Verwendung von Ultraschallsensoren an der Blasenkammer zur Ermittlung des Flüssigkeitsfüllstandes verzichtet werden.

Die Steuer- und Recheneinheit der Blutbehandlungsvorrichtung weist vorteilhaft einen Datenspeicher auf, in dem ein Computerprogramm abgespeichert ist. Der Programmcode des Computerprogramms ist programmiert zum Ansteuern des Körperschallemitters und zum Auswerten der Signale des mindestens einen Körperschallsensors. In dem Datenspeicher kann das Übertragungsverhalten bei fehlerfreiem Zustand des Abschnitts des extrakorporalen Blutkreislaufs als Referenz abgelegt werden, so dass beim Abweichen der aktuellen Messwerte von der Referenz auf einen fehlerhaften Zustand des Abschnitts geschlossen werden kann.
Ein weiterer Aspekt der Erfindung betrifft ein Verfahren zur Detektion des Füllstandes einer Blasenkammer in Fluidkreislauf eines medizinischen Gerätes, insbesondere in einem extrakorporalen Blutkreislauf in einer Blutbehandlungsvorrichtung. Bei diesem Verfahren wird über einen Körperschallemitter ein Körperschall über eine Ankoppelstelle in einen Schlauchsatz vor der Blasenkammer eingekoppelt. Der sich im Fluidkreislauf ausbreitende Körperschall wird über einen an eine in der Körperschallausbreitungsrichtung nach der Blasenkammer angeordnete Ankoppelstelle gekoppelten Körperschallsensor aufgenommen und einer Steuer- und Recheneinheit zur Bestimmung der Füllhöhe zugeleitet.

In der Steuer- und Recheneinheit wird die Füllhöhe der Flüssigkeit in der Blasenkammer vorteilhaft durch Vergleich mit abgespeicherten Kennlinien bestimmt.

Schließlich betrifft die Erfindung ein Computerprogrammprodukt mit einem Quellcode zur Ausführung des vorgenannten Verfahrens, wenn das Computerprogramm in der Steuer- und Recheneinheit abläuft.

Wie zuvor bereits ausgeführt, ist die vorliegende Erfindung zur Lösung der eingangs gestellten Aufgabe geeignet. Sie ist allerdings nicht auf die Bestimmung der Füllhöhe in Blasenkammern beschränkt. Ganz allgemein ermöglicht die Erfindung nämlich die Bestimmung des Zustandes eines Abschnitts des extrakorporalen Blutkreislaufs auf der Grundlage der Messung von Körperschall am extrakorporalen Blutkreislauf.
Neben einer Blasenkammer kann als erfindungsgemäßer Abschnitt des extrakorporalen Blutkreislaufs beispielsweise auch der Patientenzugang überwacht werden, der als Fistel oder als Shunt am Patienten angelegt sein kann.

Die vorliegende Offenbarung betrifft dabei eine Blutbehandlungsvorrichtung für die extrakorporale Blutbehandlung mit einer Steuer- und Recheneinheit, mindestens einem Körperschallemitter und mindestens einem Körperschallsensor, jeweils konfiguriert zum Ankoppeln an Ankoppelstellen eines extrakorporalen an die Blutbehandlungsvorrichtung angekoppelten Blutschlauchsatzes, wobei über die Steuer-und Recheneinheit der Zustand eines Patientenzugangs auf der Grundlage der Messung des Körperschalls am extrakorporalen Blutkreislauf überwacht wird.
Eine unbekannte venöse Nadeldiskonnektion aus dem Patientenzugang kann zum befürchteten Freifluss des Bluts aus der venösen Nadel in die Umgebung führen, falls die Blutpumpe weiterpumpt. Falls ein solcher schwerer Störfall nicht sofort erkannt wird, kann der Patient bei üblichen Blutflüssen von 200 ml/min bis 300 ml/min innerhalb von wenigen Minuten verbluten. Während eine Diskonnektion der arteriellen Nadel aufgrund der zwangsläufig aus der Umgebung in den extrakorporalen Blutkreislauf gesaugten Umgebungsluft sofort und zuverlässig durch die stets zur Verhinderung von Luftembolien vorhandenen Luftblasendetektoren erkannt wird, stellt die sichere Erkennung einer venösen Nadeldiskonnektion trotz vieler bekannter Lösungsansätze immer noch eine technische Herausforderung dar, weil gleichzeitig die Häufigkeit falscher positiver Alarme (d. h. entsprechender Fehlalarme) minimiert werden muss.

Entsprechend einem weiteren Aspekt der Offenbarung ist es dabei möglich, eine Nadeldiskonnektion, insbesondere eine arterielle und/oder venöse Nadeldiskonnektion zu delektieren, wenn sich die Schallübertragung im extrakorporalen Blutkreislauf aufgrund des fehlerhaften Patientenzugangs ändert und die gemessene Amplitude des Körperschalls abnimmt oder sogar ausbleibt. Ein solches Verfahren ist aus der WO 97/10013 A1 bekannt, bei welcher die Übertragung der durch eine okkludierende Blutpumpe erzeugten Druckpulse über den Patientenzugang überwacht wird.

Aufgabe der vorliegenden Offenbarung gegenüber dieser Druckschrift ist es, das Risiko eines Fehlalarms zu verringern und dennoch eine sichere Erfassung einer Nadeldiskonnektion zu gewährleisten.
Diese Aufgabe wird durch eine Blutbehandlungsvorrichtung und ein Verfahren gemäß folgenden Aspekten gelöst.
1. Blutbehandlungsvorrichtung für die extrakorporale Blutbehandlung mit einer Steuer- und Recheneinheit, mindestens einem Körperschallemitter und mindestens einem Körperschallsensor, jeweils konfiguriert zum Ankoppeln an Ankoppelstellen eines extrakorporalen an die Blutbehandlungsvorrichtung ankoppelbaren Blutschlauchsatzes, wobei über die Steuer-und Recheneinheit der Zustand eines Patientenzugangs auf der Grundlage der Messung des Körperschalls am extrakorporalen Blutkreislauf überwacht wird,
   dadurch gekennzeichnet,
   dass in der Steuer- und Recheneinheit zur Detektion einer Nadeldiskonnektion das aktuell gemessene Signal mit einem Basissignal verglichen wird, welches während des Behandlungsverlaufs aktualisiert wird.
2. Blutbehandlungsvorrichtung nach Aspekt 1, wobei das Basissignal durch die Steuer- und Recheneinheit in regelmäßigen Abständen und/oder laufend aktualisiert wird.
3. Blutbehandlungsvorrichtung nach Aspekt 1 oder 2, wobei das Basissignal nach einer gesteuerten Änderung des Pegels in einer Blasenkammer des extrakorporalen Blutkreislaufs aktualisierbar ist, und insbesondere nach einer Entlüftung der Blasenkammer, wobei die Aktualisierung bevorzugt durch die Steuer- und Recheneinheit automatisiert durchgeführt wird.
4. Blutbehandlungsvorrichtung nach einem der vorangegangenen Aspekte, wobei die Steuer- und Recheneinheit zur Aktualisierung des Basissignals einen zeitlichen Mittelwert des gemessenen Signals bildet.
5. Blutbehandlungsvorrichtung nach einem der vorangegangenen Aspekte, wobei der Vergleich des aktuell gemessenen Signals mit einem Basissignal einen Amplitudenvergleich umfasst, wobei bevorzugt die Amplitude des aktuell gemessenen Signals mit einem Basisamplitudenwert verglichen wird.
6. Blutbehandlungsvorrichtung nach einem der vorangegangenen Aspekte, wobei die Steuer- und Recheneinheit zum Vergleich des aktuell gemessenen Signals mit einem Basissignal ein Signalanteil des gemessenen Signals extrahiert.
7. Blutbehandlungsvorrichtung nach einem der vorangegangenen Aspekte, wobei die Steuer- und Recheneinheit eine Nadeldiskonnektion detektiert, wenn sich die Schallausbreitung im extrakorporalen Blutkreislauf aufgrund des fehlerhaften Patientenzugangs ändert und/oder die gemessene Amplitude des Körperschalls abnimmt oder ausbleibt, wobei sie bevorzugt bei Detektion einer Nadeldiskonnektion die Blutpumpe stoppt und/oder einen Alarm auslöst.
8. Blutbehandlungsvorrichtung nach einem der vorangegangenen Aspekte, wobei es sich bei dem Körperschallemitter um eine okkludierende Pumpe, insbesondere eine Rollenpumpe handelt, welche an einen Pumpabschnitt des Schlauchsatzes ankoppelbar ist, und/oder wobei es sich bei dem Körperschallemitter um einen elektronisch angesteuerten Schallerzeuger handelt, welcher bevorzugt ein Piezoelement umfasst und/oder bevorzugt stromaufwärts oder stromabwärts einer Pumpe am Schlauchsatz ankoppelbar ist.
9. Blutbehandlungsvorrichtung nach einem der vorangegangenen Aspekte mit einem Blutschlauchsatz, welcher mindestens eine Ankoppelstelle für einen Körperschallemitter und mindestens eine Ankoppelstelle für einen Körperschallsensor aufweist, wobei die Ankoppelstelle für den Körperschallemitter am arteriellen Zweig des Blutschlauchsatzes stromaufwärts der Blutpumpe angeordnet ist und/oder die Blutpumpe als Körperschallemitter dient, und/oder wobei die Ankoppelstelle für den Körperschallsensor stromabwärts oder stromaufwärts der venösen Blasenkammer angeordnet ist.
10. Verfahren zur Überwachung des Zustands eines Patientenzugangs eines extrakorporalen Blutkreislaufs einer Blutbehandlungsvorrichtung für die extrakorporale Blutbehandlung, wobei der Zustand des Patientenzugangs auf der Grundlage der Messung von Körperschall am extrakorporalen Blutkreislauf überwacht wird,
   dadurch gekennzeichnet,
   dass zur Detektion einer Nadeldiskonnektion das aktuell gemessene Signal mit einem Basissignal verglichen wird, welches während des Behandlungsverlaufs aktualisiert wird.
11. Verfahren nach Aspekt 10, wobei das Basissignal in regelmäßigen Abständen und/oder laufend aktualisiert wird und/oder wobei das Basissignal nach einer gesteuerten Änderung des Pegels in einer Blasenkammer des extrakorporalen Blutkreislaufs aktualisiert wird, und insbesondere nach einer Entlüftung der Blasenkammer und/oder wobei zur Aktualisierung des Basissignals ein zeitlicher Mittelwert herangezogen wird.
12. Verfahren nach einem der vorangegangenen Aspekte, wobei der Vergleich des aktuell gemessenen Signals mit einem Basissignal einen Amplitudenvergleich umfasst, wobei bevorzugt die Amplitude des aktuell gemessenen Signals mit einem Basisamplitudenwert verglichen wird.
13. Verfahren nach einem der vorangegangenen Aspekte, wobei zum Vergleich des aktuell gemessenen Signals mit einem Basissignal ein Signalanteil des gemessenen Signals extrahiert und mit dem Basissignal verglichen wird.
14. Verfahren nach einem der vorangegangenen Aspekte, wobei eine Nadeldiskonnektion detektiert wird, wenn sich die Schallausbreitung im extrakorporalen Blutkreislauf aufgrund des fehlerhaften Patientenzugangs ändert und/oder die gemessene Amplitude des Körperschalls abnimmt oder ausbleibt, wobei bevorzugt bei Detektion einer Nadeldiskonnektion die Blutpumpe gestoppt und/oder einen Alarm ausgelöst wird, und/oder wobei das sich durch extrakorporalen Blutkreislauf und den Patientenzugang ausbreitende Signal einer okkludierenden Pumpe, insbesondere einer Rollenpumpe gemessen wird, welche an einen Pumpabschnitt des Schlauchsatzes angekoppelt ist, und/oder wobei das sich durch den extrakorporalen Blutkreislauf und den Patientenzugang ausbreitende Signal eines elektronisch angesteuerten Schallerzeuger gemessen wird, welcher bevorzugt ein Piezoelement umfasst und/oder bevorzugt stromaufwärts oder stromabwärts einer Pumpe am Schlauchsatz angekoppelt ist, und/oder wobei der Körperschallemitter am arteriellen Zweig des Blutschlauchsatzes stromaufwärts der Blutpumpe angekoppelt ist und/oder die Blutpumpe als Körperschallemitter dient, und/oder wobei der Körperschallsensor stromabwärts oder stromaufwärts der venösen Blasenkammer angekoppelt ist.
15. Verfahren nach einem der vorangegangenen Aspekte zur Überwachung des Zustands eines Patientenzugangs eines extrakorporalen Blutkreislaufs einer Blutbehandlungsvorrichtung gemäß einem der Aspekte 1 bis 9.

Die vorliegende Offenbarung umfasst dabei eine Blutbehandlungsvorrichtung für die extrakorporale Blutbehandlung mit einer Steuer- und Recheneinheit, mindestens einem Körperschallemitter und mindestens einem Körperschallsensor, jeweils konfiguriert zum Ankoppeln an Ankoppelstellen eines extrakorporalen an die Blutbehandlungsvorrichtung ankoppelbaren Blutschlauchsatzes. Dabei kann über die Steuer- und Recheneinheit der Zustand eines Patientenzugangs auf der Grundlage der Messung des Körperschalls am extrakorporalen Blutkreislauf überwacht werden. Bei dem Patientenzugang kann es sich um eine Fistel oder einen Schunt handeln, über welchen der extrakorporale Blutschlauchsatz mit dem Patienten verbunden ist. Beispielhaft vergleicht die Steuer- und Recheneinheit dabei zur Detektion einer Nadeldiskonnektion das aktuell gemessene Signal mit einem Basissignal, welches während des Behandlungsverlaufs aktualisiert wird.

Die vorliegende Offenbarung basiert dabei auf der Erkenntnis, dass das am Körperschallsensor gemessene Körperschallsignal nicht nur durch den Zustand des Patientenzugangs beeinflusst wird, sondern auch durch Vorgänge im Blutschlauchsatz selbst. Untersuchungen haben dabei überraschend gezeigt, dass sich der Körperschall nicht nur über die Flüssigkeit im Blutschlauchsatz fortpflanzt, sondern maßgeblich auch über längere Strecken in den Wandungen des Schlauches und in den Wandungen der Komponenten des Blutschlauchsatzes weitergeleitet wird, und insbesondere auch in den Wandungen einer Blasenkammer. Dabei wirken die Wandungen der Bauteile jedoch im Vergleich zur Schallleitung der Flüssigkeit im Blutschlauchsatz eher dämpfend auf die Schallausbreitung. Die vorliegende Erfindung berücksichtigt daher, dass die Schallausbreitung im extrakorporalen Blutschlauchsatz in Abhängigkeit von dem Füllstand der dort eingesetzten Komponenten, und insbesondere in Abhängigkeit von dem Füllstand einer im extrakorporalen Blutschlauchsatz angeordneten Blasenkammer sich während der Behandlungsdauer verändern kann.
Die erfindungsgemäße Aktualisierung des Basissignals, mit welchem das aktuell gemessene Signal zur Detektion einer Nadeldiskonnektion verglichen wird, sorgt nun dafür, dass solche sich während des Behandlungsverlaufs ergebenden Änderungen im Übertragungsverhalten des Blutschlauchsatzes berücksichtigt werden und so weder einen Fehlalarm auslösen, noch die Sicherheit der Detektion einer Nadeldiskonnektion negativ beeinflussen.

Gemäß einer möglichen Ausführungsform der vorliegenden Erfindung wird das Basissignal dabei durch die Steuer- und Recheneinheit in regelmäßigen Abständen und / oder laufend aktualisiert. Da die oben beschriebenen Veränderungen im Blutschlauchsatz wie beispielsweise die Füllhöhe einer Blasenkammer sich üblicherweise kontinuierlich und relativ langsam verändern, wird hierdurch sichergestellt, dass das für die Detektion einer Nadeldiskonnektion herangezogene Basissignal den aktuellen Zustand des Blutschlauchsatzes gut wiedergibt.

Weiterhin kann vorgesehen sein, dass das Basissignal nach einer gesteuerten Änderung des Pegels in einer Blasenkammer des extrakorporalen Blutkreislaufs aktualisierbar ist. Insbesondere kann das Basissignal dabei nach einer Entlüftung der Blasenkammer aktualisierbar sein. Bei der Entlüftung einer Blasenkammer steigt der Pegel innerhalb der Blasenkammer relativ schnell, was zu einer entsprechend verbesserten Schallübertragung über die Blasenkammer und damit einer Erhöhung des Signalpegels führt. Dies wird erfindungsgemäß durch eine nach einer solchen Änderung des Pegels vorgenommenen Aktualisierung berücksichtigt.

Bevorzugt führt die Steuer- und Recheneinheit dabei die Aktualisierung automatisch durch, nachdem die gesteuerte Pegeländerung durchgeführt wurde. Bevorzugt wird dabei auch die gesteuerte Pegeländerung und insbesondere die Entlüftung automatisch durch die Steuer- und Recheneinheit durchgeführt.

Bevorzugt erzeugt die Steuer- und Recheneinheit das Basissignals dabei auf Grundlage des durch den Körperschallsensor erzeugten Signals, d.h. auf Grundlage des Signals des selben Sensors, dessen aktuelles Signal mit dem Basissignal verglichen wird.

Dabei kann erfindungsgemäß vorgesehen sein, dass die Steuer- und Recheneinheit zur Aktualisierung des Basissignals einen zeitlichen Mittelwert des gemessenen Signals bildet. Ein solcher zeitlicher Mittelwert sorgt dafür, dass kurzzeitige Schwankungen des Signals bei der Bildung des Basissignals unberücksichtigt bleiben. Hierdurch wird wiederum die Sicherheit einer Detektion verbessert.

Dieser zeitliche Mittelwert des gemessenen Signals wird dann gemäß der vorliegenden Erfindung während des Behandlungsverlaufs aktualisiert, bspw. in regelmäßigen Abständen oder laufend. Hierzu kann in die Bildung des zeitlichen Mittelwertes jeweils der Signalverlauf in einer bestimmten, der Aktualisierung vorangehenden Zeitspanne eingehen.

Der Vergleich des aktuell gemessenen Signals mit einem Basissignal, welcher zur Detektion einer Nadeldiskonnektion eingesetzt wird, kann dabei erfindungsgemäß einen Amplitudenvergleich umfassen. Insbesondere kann dabei die Amplitude des aktuell gemessenen Signals mit einem Basisamplitudenwert verglichen werden. Insbesondere kann die Steuer- und Recheneinheit dabei vor Beginn der Behandlung und / oder während des Behandlungsverlaufs einen Basisamplitudenwert bilden und abspeichern, mit welchem die aktuell gemessene Amplitude verglichen wird. Alternativ kann auch das Basissignal abgespeichert und jeweils ein Basisamplitudenwert aus dem Basissignal erzeugt werden um mit dem aktuellen Amplitudenwert verglichen zu werden.

Gemäß einem Aspekt der vorliegenden Erfindung kann zum Vergleich des aktuell gemessenen Signals mit einem Basissignal ein Signalanteil des gemessenen Signals extrahiert werden. Dieser extrahierte Signalanteil wird dann bevorzugt mit dem Basissignal oder einem aus dem Basissignal extrahierten Signal verglichen. Insbesondere wenn mehrere Schallemitter eingesetzt werden, kann dabei das von einem dieser Emitter stammende Signal extrahiert werden, um anhand dieses Signalanteils eine Nadeldiskonnektion zu detektieren.

Erfindungsgemäß kann die Steuer- und Recheneinheit dabei eine Nadeldiskonnektion detektieren, wenn sich die Schallausbreitung im extrakorporalen Blutkreislauf aufgrund des fehlerhaften Patientenzugangs ändert und / oder die gemessene Amplitude des Körperschalls abnimmt oder ausbleibt.

Bevorzugt stoppt die Steuer- und Recheneinheit dabei bei Detektion einer Nadeldiskonnektion die Blutpumpe und / oder löst einen Alarm aus.

Gemäß einer ersten möglichen Ausführungsform der vorliegenden Erfindung kann es sich bei dem Körperschallemitter um eine okkludierende Pumpe handeln, welche an einem Pumpabschnitt des Schlauchsatzes ankoppelbar ist. Solche okkludierenden Pumpen erzeugen während ihres Betriebes starke Drucksignale, welche sich als Körperschall durch den extrakorporalen Blutkreislauf ausbreiten. Insbesondere kann es sich bei der okkludierenden Pumpe dabei um eine Rollenpumpe handeln.

In einer weiteren möglichen Ausführungsform kann es sich bei dem Körperschallemitter auch um einen elektronisch angesteuerten Schallerzeuger handeln. Dieser kann beispielsweise stromaufwärts oder stromabwärts einer Pumpe am Schlauchsatz angekoppelt sein. Der elektronisch angesteuerte Schallerzeuger wandelt dabei ein elektronisches Signal in ein Schallsignal um. Der Schallerzeuger kann dabei insbesondere ein Piezoelement umfassen. Bei der Pumpe kann es sich in diesem Fall auch um eine nicht-okkludierende Pumpe handeln, insbesondere um eine Impellerpumpe. In diesem Fall breiten sich auch die von dem elektronisch angesteuerten Schallerzeuger erzeugten Schallwellen über zwei Ausbreitungspfade aus.

Gemäß einer möglichen Ausführungsform der vorliegenden Erfindung kann dabei ein einziger Körperschallemitter eingesetzt werden, dessen Signal sich über den extrakorporalen Blutschlauchsatz und den Patientenzugang bis zum Körperschallsensor ausbreitet. Das Signal kann sich dabei in zwei Wegen, zum einen über den extrakorporalen Blutschlauchsatz, und zum anderen über den Patientenzugang zum Körperschallsensor ausbreiten. Dabei kann sich der Körperschall insbesondere auf einem ersten Weg über den extrakorporalen Blutschlauchsatz durch eine Blasenkammer zum Körperschallsensor ausbreiten, und zum anderen auf einem zweiten Weg über den Patientenzugang zum Körperschallsensor, ohne durch die Blasenkammer zu gehen.

Die erfindungsgemäße Aktualisierung des Basissignals berücksichtigt bei dieser Ausführungsform den Einfluss des Füllstands der Blasenkammer auf den Signalanteil, welcher über den ersten Weg zum Körperschallsensor gelangt.

Das Signal kann sich aber auch in nur einem Weg über den den Patientenzugang zum Körperschallsensor ausbreiten. Dabei kann der Körperschall insbesondere die Blasenkammer passieren, bevor oder nachdem er den Patientenzugang passiert, sodass der Füllstand der Blasenkammer das komplette durch den Patientenzugang sich ausbreitende Signal beeinflusst.

Erfindungsgemäß können aber auch zwei Körperschallemitter mit unterschiedlichen Signalverläufen eingesetzt werden, insbesondere eine okkludierende Blutpumpe und ein stromaufwärts oder stromabwärts der Blutpumpe angeordneter weiterer Körperschallemitter.

Bevorzugt werden dabei die auf die beiden Körperschallemitter entfallenden Signalanteile getrennt, und zur Überwachung des Patientenzugangs zumindest der auf dem näher am Patientenzugang angeordneten Körperschallemitter beruhende Signalanteil herangezogen. Insbesondere kann dabei das Signal des Körperschallemitters, welches in der arteriellen Leitung näher am Patienten angeordnet ist, und damit sein Signal unmittelbar über den Patientenzugang zur venösen Leitung leitet, herangezogen werden.

Als Schallgenerator kann prinzipiell eine okkludierende Blutpumpe verwendet werden, die stromaufwärts und stromabwärts starke Druckpulse erzeugt, sowie dies in der WO 97/10013 A1 vorgeschlagen wird.

Als zuverlässiger hat sich jedoch erwiesen, ein separates Körperschallsignal mit definierter Frequenz einzuspeisen. Als besonders geeignet für die Einspeisung in den extrakorporalen Blutschlauchsatz hat sich eine sinusförmige Frequenz im Frequenzbereich von mindestens 80 Hz, insbesondere 160 Hz erwiesen. Auch ein rechteckiges Pulssignal ist geeignet.

An einem extrakorporalen Blutkreislauf kann im Falle einer venösen Nadeldiskonnektion eine Verringerung der gemessenen Amplitude des Körperschalls um rund 30 % erwartet werden, so dass in diesem Fehlerfall ein signifikanter Hinweis auf eine venöse Nadeldiskonnektion vorliegt. Insbesondere schließt die Steuer- und Recheneinheit daher auf eine Nadeldiskonnektion, wenn eine Verringerung der gemessenen Amplitude des Körperschalls um einen Schwellwert, welcher zwischen 20 % und 60 % des Basissignals liegt, erkannt wird.

Als Körperschallsensor ist beispielsweise ein Piezofoliensensor geeignet, der an den extrakorporalen Blutschlauchsatz ankoppelbar ist.

Die vorliegende Erfindung umfasst dabei weiterhin eine Blutbehandlungsmaschine mit einem Blutschlauchsatz, wie sie oben beschrieben wurden, wobei der Blutschlauchsatz mindestens eine Ankoppelstelle für einen Körperschallemitter und mindestens eine Ankoppelstelle für einen Körperschallsensor aufweist. Vorteilhafterweise ist die Ankoppelstelle für den Körperschallemitter dabei am arteriellen Zweig des Blutschlauchsatzes stromaufwärts der Blutpumpe angeordnet, oder die Blutpumpe selbst dient als Körperschallemitter.

Dabei kann die Ankoppelstelle für den Körperschallsensor stromabwärts der venösen Blasenkammer angeordnet sein. Breitet sich der Körperschall ausgehend von dem Körperschallemitter nun sowohl in dem arteriellen, wie auch in dem venösen Zweig des extrakorporalen Blutschlauchsatzes gleichzeitig aus, so treffen Schallwellen auf den Körperschallsensor auf, welche die Blasenkammer durchlaufen haben, und andere, welche nur den Patientenzugang durchlaufen haben.

In einer alternativen Ausführungsform ist die Ankoppelstelle für den Körperschallsensor stromaufwärts der venösen Blasenkammer angeordnet. In diesem Fall haben die Schallwellen, welche sich vom Körperschallemitter über den Patientenzugang in die venöse Leitung ausbreiten, vor dem Eintreffen bei dem Körperschallemitter die Blasenkammer durchlaufen. Das gemessene Signal umfasst damit Signalanteile, welche auf dem Durchlaufen der Blasenkammer beruhen, und Signalanteile, welche auf dem Durchlaufen des Patientenzugangs beruhen. Durch die Aktualisierung des Basissignals können jedoch die aufgrund der Pegeländerung in der Blasenkammer hervorgerufenen Änderungen im Signal bei der Detektion einer Nadeldiskonnektion berücksichtigt werden.

Die vorliegende Erfindung umfasst weiterhin ein Verfahren zur Überwachung des Zustands eines Patientenzugangs eines extrakorporalen Blutkreislaufs einer Blutbehandlungsvorrichtung für die extrakorporale Blutbehandlung, wobei der Zustand des Patientenzugangs auf der Grundlage der Messung von Körperschall am extrakorporalen Blutkreislauf überwacht wird. Erfindungsgemäß wird dabei zur Detektion einer Nadeldiskonnektion das aktuell gemessene Signal mit einem Basissignal verglichen, welches während des Behandlungsverlaufs aktualisiert wird. Auch hierdurch kann der Einfluss des Übertragungsverhaltens des Blutschlauchsatzes und insbesondere der Einfluss der Füllhöhe einer Blasenkammer auf die Schallausbreitung im Blutschlauchsatz bei der Detektion einer Nadeldiskonnektion berücksichtigt werden.

Bevorzugt wird dabei das Basissignal in regelmäßigen Abständen und / oder laufend aktualisiert. Weiterhin kann vorgesehen sein, dass das Basissignal nach einer gesteuerten Änderung des Pegels in einer Blasenkammer des extrakorporalen Blutkreislaufs aktualisiert wird. Insbesondere kann dies nach einer Entlüftung der Blasenkammer erfolgen.

Weiterhin kann gemäß dem erfindungsgemäßen Verfahren zur Aktualisierung des Basissignals ein zeitlicher Mittelwert herangezogen werden.

Der erfindungsgemäße Vergleich des aktuell gemessenen Signals mit einem Basissignal kann einen Amplitudenvergleich umfassen. Bevorzugt wird dabei die Amplitude des aktuell gemessenen Signals mit einem Basisamplitudenwert verglichen.

Weiterhin kann zum Vergleich des aktuell gemessenen Signals mit einem Basissignal ein Signalanteil des gemessenen Signals extrahiert und mit einem Basissignal verglichen werden. Insbesondere kann dabei ein Signalanteil, welcher auf einem von mehreren Körperschallemittern basiert, extrahiert werden.

Erfindungsgemäß kann dabei eine Nadeldiskonnektion detektiert werden, wenn sich die Schallausbreitung im extrakorporalen Blutkreislauf aufgrund des fehlerhaften Patientenzugangs ändert und / oder die gemessene Amplitude des Körperschalls abnimmt oder ausbleibt. Bevorzugt wird dabei bei Detektion einer Nadeldiskonnektion die Blutpumpe gestoppt und / oder ein Alarm ausgelöst. Insbesondere kann erfindungsgemäß eine venöse Nadeldiskonnektion detektiert werden.

Gemäß der vorliegenden Erfindung kann in einer ersten möglichen Variante das sich durch den extrakorporalen Blutkreislauf und den Patientenzugang ausbreitende Signal einer okkludierenden Pumpe gemessen werden. Insbesondere kann es sich dabei um das Signal einer Rollenpumpe handeln. Die okkludierende Pumpe ist dabei an einen Pumpabschnitt des Schlauchsatzes angekoppelt und erzeugt Druckpulse, welche sich als Körperschallsignal ausbreiten.

Alternativ oder zusätzlich kann das Signal eines elektronisch angesteuerten Schallerzeugers gemessen werden. Der Schallerzeuger kann dabei ein Piezoelement umfassen. Bevorzugt wird der Schallerzeuger stromaufwärts oder stromabwärts einer Pumpe am Schlauchsatz angekoppelt. Insbesondere kann dabei der elektronisch angesteuerte Schallerzeuger mit einem Ansteuersignal angesteuert werden, und dieses in ein Schallsignal umwandeln. Bevorzugt unterscheidet sich das durch den elektronisch angesteuerten Schallerzeuger erzeugte Schallsignal von dem durch die Pumpe erzeugten Signal.

Erfindungsgemäß können dabei sowohl eine okkludierende Pumpe, als auch ein elektronisch angesteuerter Schallerzeuger als Körperschallemitter eingesetzt werden. Alternativ kann ein elektronisch angesteuerter Schallerzeuger jedoch auch zusammen mit einer nicht-okkludierenden Pumpe, und insbesondere einer Impellerpumpe eingesetzt werden.

Gemäß der vorliegenden Erfindung kann der Körperschallemitter am arteriellen Zweig des Blutschlauchsatzes und insbesondere stromaufwärts der Blutpumpe angekoppelt sein, und/oder die Blutpumpe als Körperschallemitter dienen. Der Körperschallsensor kann stromabwärts oder stromaufwärts der venösen Blasenkammer angekoppelt sein.

Bevorzugt wird das erfindungsgemäße Verfahren dabei so durchgeführt, wie dies bereits oben im Hinblick auf die Funktionsweise der erfindungsgemäßen Blutbehandlungsvorrichtung näher dargestellt wurde. Insbesondere kann das erfindungsgemäße Verfahren dabei dazu eingesetzt werden, um den Zustand eines Patientenzugangs eines extrakorporalen Blutkreislaufes einer Blutbehandlungsvorrichtung, wie sie oben dargestellt wurde, zu überwachen.

Weitere Merkmale, Einzelheiten und Vorteile der Erfindung ergeben sich aus der folgenden Beschreibung der zeichnerisch dargestellten Ausführungsbeispiele. Es zeigen:
- Figur 1:: die schematische Darstellung eines extrakorporalen Blutkreislaufs mit beispielhaften Positionen eines Körperschallemitters und eines Körperschallsensors;
- Figur 2:: eine schematische Darstellung der Ausbreitung des von der Blutpumpe in einen extrakorporalen Blutkreislauf erzeugten Körperschalls entlang des arteriellen und des venösen Zweigs des extrakorporalen Blutkreislaufs bis zum Auftreffen auf einen Körperschallsensor stromabwärts der Blutpumpe;
- Figur 3:: die Amplitude des gemessenen Körperschalls nach Durchlaufen einer venösen Blasenkammer eines extrakorporalen Blutkreislaufs, gemäß Figur 1 in Abhängigkeit vom Füllstand des Bluts in der Blasenkammer und
- Figur 4:: Messwerte der relativen Abnahme des gemessenen Körperschalls nach Durchlaufen eines extrakorporalen Blutkreislaufs und des Patientenzugangs gemäß Figur 1 in Abhängigkeit von der Zeit bei Auftreten einer Nadeldiskonnektion nach einem Zeitpunkt von t = 8 Sek.,
- Figur 5:: die schematische Darstellung eines extrakorporalen Blutkreislaufs mit alternativen beispielhaften Positionen eines Körperschallemitters und eines Körperschallsensors.

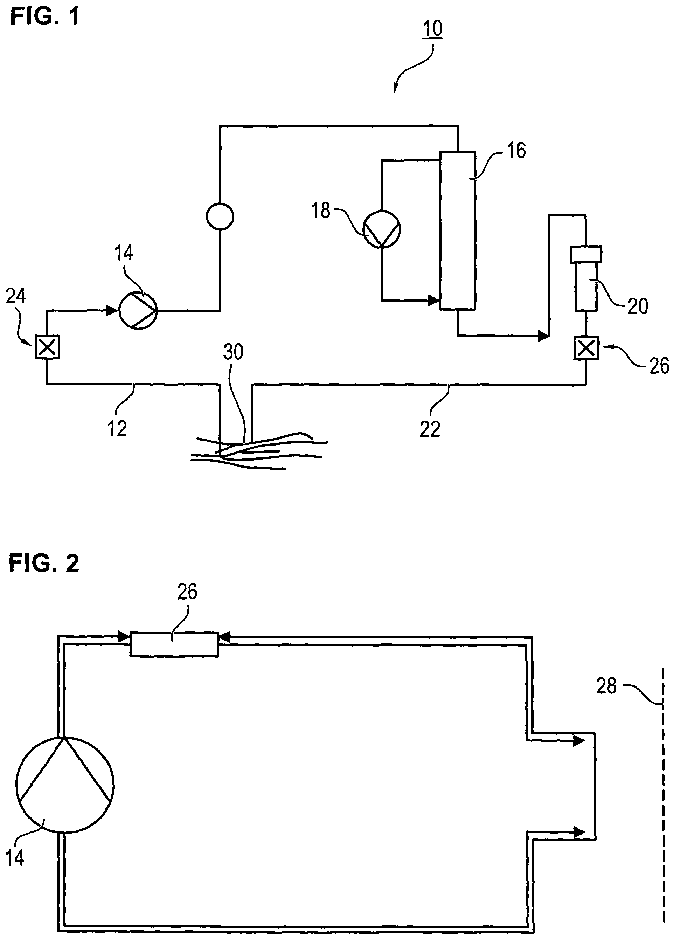

Figur 1 zeigt ganz allgemein in schematischer Darstellung einen extrakorporalen Blutkreislauf 10, bei dem ein arterieller Schlauch 12 mit einer Arterie eines Patienten in Verbindung steht. Hier ist in üblicherweise eine Blutpumpe 14 und ein Dialysator 16 gezeigt. Parallel zum Dialysator ist eine Dialysatpumpe 18 dargestellt. Dem Dialysator nachgeschaltet ist eine Blasenkammer 20. An diese schließt ein venöser Schlauch 22 an, der wieder mit einer entsprechenden Vene des Patienten konnektiert ist.

In dem extrakorporalen Blutkreislauf 10 ist nun ein Körperschallemitter 24 und ein Körperschallsensor 26 angeordnet. Diese dienen dazu, mit einer hier nicht näher dargestellten Steuer- und Recheneinheit die Ausbreitung des Körperschalls im extrakorporalen Blutschlauchsatz zu bestimmen. Der Körperschall kann beispielsweise mittels einer Schallquelle an der Blutbehandlungsvorrichtung in den Blutschlauchsatz eingekoppelt werden. Eine geeignete Ankoppelstelle für den Körperschall kann im arteriellen Zweig des Blutschlauchsatzes stromaufwärts der Blutpumpe, d. h. entsprechend dem hier dargestellten Ausführungsbeispiel ca. 30 cm stromaufwärts der Blutpumpe erfolgen. Der Körperschallemitter 24 ist also ca. 30 cm stromaufwärts der Blutpumpe 14 angeordnet (vgl. Figur 1).

Eine mögliche Position eines Körperschallsensors 26 kann, wie in Figur 1 ebenfalls dargestellt, stromabwärts der venösen Blasenkammer 20 vorgesehen sein. Ab der gewünschten Sensorposition weist die Länge des venösen Schlauchs 22 noch ca. 1,6 m Länge auf. Der Körperschallemitter und der Körperschallsensor können insbesondere in der Blutbehandlungsvorrichtung integriert sein, so dass diese beim Einlegen eines extrakorporalen Blutschlauchsatzes jeweils an den vorgesehenen Ankoppelstellen an den extrakorporalen Blutschlauchsatz angekoppelt werden können. Dies ist in der schematischen Darstellung gemäß Figur 1 nicht im Einzelnen dargestellt.

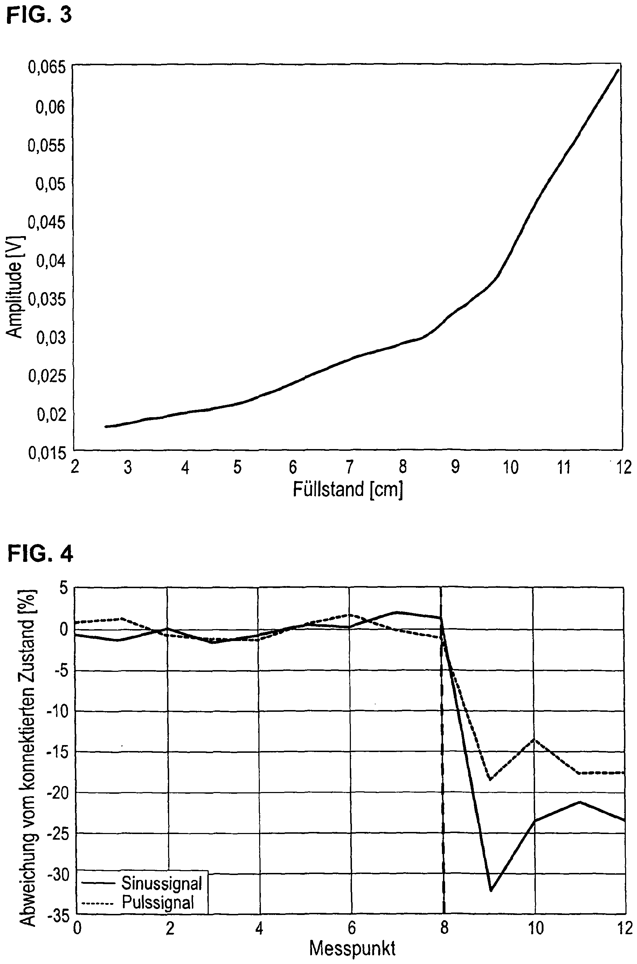

Die Wandungen der Bauteile des extrakorporalen Blutkreislaufs 10 wirken im Vergleich zur Schallleitung der Flüssigkeit im Blutschlauchsatz eher dämpfend auf die Schallausbreitung. Es hat sich gezeigt, dass sich das Dämpfungsverhalten der venösen Blasenkammer 20 signifikant und reproduzierbar mit abnehmendem Füllstand verstärkt. Je höher der Füllstand ist, desto geringer ist die Dämpfung. Die vorliegende Erfindung beruht daher darauf, dass vom gemessenen Dämpfungsverhalten der Blasenkammer 20 auf deren Füllstand geschlossen wird. Das Dämpfungsverhalten wird ermittelt, indem die Amplitude des Körperschalls in einer Schallausbreitungseinrichtung hinter der zu vermessenden Blasenkammer gemessen wird, wobei der gemessene Körperschall beim Durchlaufen der Blasenkammer 20 in Abhängigkeit vom Füllstand unterschiedlich stark gedämpft wird. In der Figur 3 ist die gemessene Amplitude des Körperschalls in Abhängigkeit vom Füllstand einer beispielhaften Blasenkammer 20 dargestellt, wobei in der Richtung des Blutflusses stromabwärts der Blasenkammer die Amplitude des Körperschalls gemessen wird. Die gemessene Amplitude des Körperschalls nimmt bei der beispielhaften Blasenkammer mit zunehmendem Füllstand der Blasenkammer progressiv zu, weil die Dämpfung der Schallwellen entsprechend abnimmt. Eine entsprechende Kennlinie kann in der hier nicht näher dargestellten Steuer- und Recheneinheit der Blutbehandlungsmaschine abgespeichert werden, so dass aus aktuellen Messwerten der Amplitude des Körperschalls der Füllstand in der Blasenkammer berechnet werden kann.

In Figur 2 ist hier ganz schematisch die Ausbreitung des von der Blutpumpe in einem extrakorporalen Blutkreislaufs erzeugten Körperschalls entlang des arteriellen und des venösen Zweigs des extrakorporalen Blutkreislaufs bis zum Auftreffen auf einen Körperschallsensor stromabwärts der Blutpumpe dargestellt. Als Sensor 26 kann hier ein Piezofoliensensor eingesetzt werden. Mit der gestrichelten Linie ist ein nicht näher dargestellter Shunt 28 ganz allgemein dargestellt.

Gemäß einem weiteren Aspekt der Erfindung kann auch eine Nadeldiskonnektion, insbesondere eine arterielle und/oder venöse Nadeldiskonnektion detektiert werden, wenn sich die Schallübertragung im extrakorporalen Blutkreislauf aufgrund des fehlerhaften Patientenzugangs ändert und die gemessene Amplitude des Körperschalls abnimmt oder sogar ausbleibt. An einem extrakorporalen Blutkreislauf kann im Falle einer venösen Nadeldiskonnektion (vgl. Figur 1 und hier insbesondere die Bezugsziffer 30) eine Verringerung der gemessenen Amplitude des Körperschalls um rund 30 % erwartet werden, so dass in diesem Fehlerfall ein signifikanter Hinweis auf eine venöse Nadeldiskonnektion vorliegt, wie dies anhand der Darstellung der Figur 4 deutlich wird. Hier ist mit gestrichelter Linie die Nadeldiskonnektion nach einer Zeit t = 8 Sek. dargestellt.

Gemäß der vorliegenden Erfindung können dabei die Detektion einer Nadeldiskonnektion und die Füllstandsmessung in der Blasenkammer miteinander kombiniert werden, oder der Einfluss der Schallleitfähigkeit des Blutschlauchsatzes bei der Detektion einer Nadeldiskonnektion berücksichtigt werden.

Erfindungsgemäß wurde dabei erkannt, dass sich das Signal des Körperschallemitters auch über die Schlauchleitung bzw. Wandung der Blasenkammer überträgt und so nicht nur das Messsignal jenseits von Blutpumpe, Dialysator und Blasenkammer beeinflusst, sondern sogar zusätzlich eine Bestimmung des Pegelstandes in der Blasenkammer ermöglicht.

Erfindungsgemäß kann daher zusätzlich zu der Pegelbestimmung in der Blasenkammer simultan der Zustand des Patientenzugangs und insbesondere die arterielle und / oder venöse Konnektion des Blutschlauchsatzes an den Patienten überwacht werden.

Bei dem in Figuren 1 bzw. 2 dargestellten Ausführungsbeispiel läuft der Signalweg über den Patientenzugang 30 nicht durch die Blasenkammer 20, da diese vom Patientenzugang kommend in der venösen Leitung hinter dem Körperschallsensor 26 liegt. Die Stärke des vom Körperschallemitter 24 über den Patientenzugang 30 zum Körperschallsensor 26 gelangenden Signals hängt daher nicht vom Pegelstand in der Blasenkammer 20 ab.

Allerdings kann es sich bei der Pumpe 14 in einer ersten Ausführungsform um eine nicht-okkludierende Pumpe handeln, so dass das Signal des Körperschallemitters 24 auch über den extrakorporalen Blutkreislauf und insbesondere den Dialysator 16 und die Blasenkammer 20 zum Körperschallsensor 26 gelangt. Das am Körperschallsensor 26 vorliegende Signal ist damit eine Überlagerung des durch den Patientenzugang 30 laufenden Signalanteils, und des durch die Blasenkammer 20 laufenden Signalanteils. Da der durch die Blasenkammer laufende Signalanteil von dem Pegelstand der Blasenkammer abhängt, hängt somit auch das Gesamtsignal, welches von dem Körperschallsensor 26 gemessen wird, von dem Pegelstand ab.

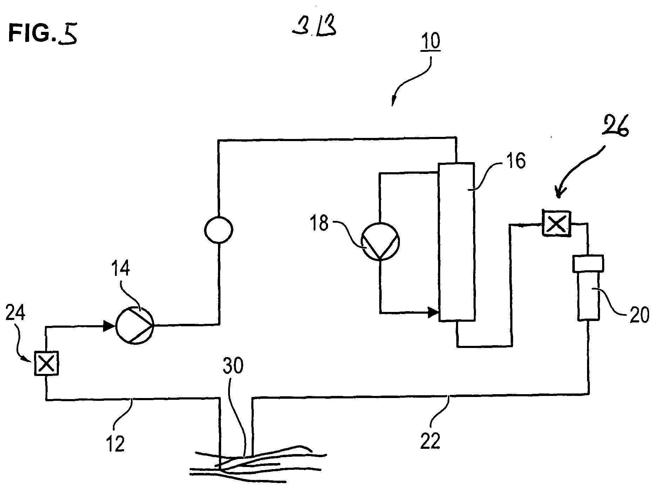

Gleiches gilt für das in Figur 5 gezeigte Ausführungsbeispiel, bei welchem der Körperschallsensor 26 zwischen dem Dialysator 16 und der venösen Blasenkammer 20 angeordnet ist, und damit vom Patientenzugang aus gesehen hinter der venösen Blasenkammer 20. Bei diesem Ausführungsbeispiel läuft daher das gesamte Signal, welches vom Körperschallemitter 24 über den Patientenzugang 30 zum Körperschallsensor 26 verläuft, durch die Blasenkammer, sodass dessen Stärke von dem Füllstand der Blasenkammer abhängt.

Je nachdem, ob es sich bei der Pumpe 14 um eine okkludierende oder um eine nicht-okkludierende Pumpe handelt, gelangt dabei bei dem in Fig. 5 gezeigten Ausführungsbeispiel auch ein Signalanteil des von dem Körperschallemitter 24 ausgesendeten Signals über den Dialysator 16 zum Körperschallsensor 26. Dieser Signalanteil wird jedoch zumindest nicht über den Pegelstand in der venösen Blasenkammer 20 beeinflusst. Gegebenenfalls ist hier jedoch eine arterielle Blasenkammer vorgesehen, deren Pegelstand dann diesen Signalanteil beeinflussen würde.

Wird dagegen in Fig. 5 eine okkludierende Pumpe 14 eingesetzt, gelangt der vom Körperschallemitter 24 erzeugte Körperschall beinahe ausschließlich über den Patientenzugang 30 zum Körperschallsensor 26, da die okkludierende Pumpe 14 die Übertragung des Körperschalls vom Körperschallemitter 24 blockiert.

Jedenfalls hat aber bei den oben dargestellten Ausführungsbeispielen die Füllhöhe in der Blasenkammer einen Einfluss auf das am Körperschallsensor 26 ankommende Signal des Körperschallemitters 24. Die gleiche Situation ergibt sich auch, wenn in weiteren alternativen Ausführungsbeispielen statt eines separaten Körperschallemitters 24 eine okkludierende Blutpumpe 14 als Körperschallemitter eingesetzt wird. Unabhängig davon, ob der Körperschallsensor 26 dann stromaufwärts oder stromabwärts der venösen Blasenkammer 20 angeordnet ist, gelangt nämlich immer einer der beiden Signalanteile, welcher von der okkludierenden Pumpe ausgestrahlt wird, über die Blasenkammer 20 zum Körperschallsensor 26.

Gemäß der vorliegenden Erfindung ist es jedoch möglich, aus dem Signal des Körperschallsensors 26 sowohl den Pegelstand in der Blasenkammer 20 zu bestimmen, als auch den Patientenzugang 30 zu überwachen. Die Messung basiert dabei im Wesentlichen auf der Erkenntnis, dass die gemessene Amplitude zwar abhängig von den Pegelständen in der Blasenkammer sinkt oder steigt, was jedoch kontinuierlich und mit deutlich niedriger Frequenz und Stärke erfolgt als der Amplitudensprung, welcher bei einer Nadeldiskonnektion, insbesondere einer venösen Nadeldiskonnektion, auftritt. Hierdurch können die beiden Vorgänge durch entsprechende Auswertung des Signals gut voneinander unterschieden werden.

Um die starke Amplitudenänderung einer Nadeldiskonnektion zu detektieren, wird eine Baseline bzw. ein Basissignal bestimmt und während des Betriebs der Blutbehandlungsvorrichtung nachgezogen. Erfindungsgemäß wird die Baseline dabei aus dem von dem Körperschallsensor 26 erzeugten Signal ermittelt. Bevorzugt gibt die Baseline durch die Aktualisierung die langfristige Änderung des Signals wieder, und stellt damit insbesondere einen zeitlichen Mittelwert dar.

Diese Baseline wird dabei in regelmäßigen Abständen sowie bei gesteuerten Pegeländerungen in der Tropfkammer aktualisiert. Die Baseline kann dabei auch laufend aktualisiert werden, bspw. indem sie als ein zeitlicher Mittelwert über einen jeweils vorangegangenen Zeitraum vorgegebener Dauer, beispielsweise über die letzten 10 bis 20 Sek., bestimmt wird.

Die Konnektion des Patienten wird überwacht, indem das aktuell von dem Körperschallsensor 26 gemessene Signal mit der Baseline verglichen wird. Kommt es hier zu einem starken Abfall des aktuell gemessenen Signals unter die Baseline, beispielsweise zu einem Abfall von mehr als 30 %, wird hieraus auf eine Nadeldiskonnektion geschlossen. Die Blutbehandlungsvorrichtung stoppt daraufhin die Blutpumpe und löst gegebenenfalls Alarm aus.

Aus der Baseline kann weiterhin der Pegelstand in der Tropfkammer bestimmt werden. Insbesondere kann dabei anhand des aktuellen Werts der Baseline über eine in der Steuerung abgespeicherte Kennlinie der Pegelstand ermittelt werden.

Die der vorliegenden Erfindung zugrunde liegende Erkenntnis, dass der Füllstand in der Blasenkammer und insgesamt der Zustand des extrakorporalen Blutschlauchsatzes sich auf das von dem Körperschallsensor 26 gemessene Signal auswirkt, sodass sich das gemessene Signal auch bei korrekter Konnektion des Patienten während der Behandlung verändern kann, kann aber auch ohne eine Bestimmung des Pegelstandes in der Blasenkammer erfindungsgemäß eingesetzt werden, um die Detektion einer Nadeldiskonnektion zu verbessern.

Insbesondere kann auch hier wie oben beschrieben eine Baseline bestimmt werden, und zur Überwachung der Konnektion das aktuell gemessene Signal mit dieser Baseline verglichen werden. Erfindungsgemäß wird die Baseline dabei während des Betriebes des Blutbehandlungsgerätes aktualisiert, um so die Detektion einer Nadeldiskonnektion robust gegen Änderungen am Blutschlauchsatz wie beispielsweise einer Änderung des Pegelstandes der Blasenkammer zu machen.

Die Baseline kann dabei insbesondere so bestimmt werden, wie dies oben beschrieben wurde. Insbesondere wird die Baseline dabei aus dem Signal des Körperschallsensors 26 gebildet, dessen Signal auch zur Detektion der Diskonnektion durch Vergleich mit der Baseline herangezogen wird. Das Signal kann dabei in regelmäßigen Abständen und / oder nach einer gesteuerten Pegeländerung in der Blasenkammer aktualisiert werden, d. h. insbesondere dann, wenn durch Entlüften der Blasenkammer der Pegel ansteigt. Als Baseline kann dabei ein über die Zeit gemitteltes Signal eingesetzt werden. Gegebenenfalls kann die Baseline dabei auch laufend aktualisiert werden, indem jeweils ein über einen gewissen vorangegangenen Zeitraum gemitteltes Signal als Baseline herangezogen wird. Zur Detektion einer Nadeldiskonnektion wird demgemäß bei dieser Ausführungsvariante der aktuelle Signalwert mit einem Mittelwert des Signals über einen jeweils vorangegangenen Zeitraum von vorgegebener Dauer verglichen.

Die Auswertung kann dabei anhand der Amplitude des Signals erfolgen. Gegebenenfalls kann der Auswertung jedoch auch eine Signalaufbereitung vorausgehen, beispielsweise die Extraktion eines bestimmten Frequenzbereiches, um so das von dem Körperschallemitter ausgesandte Signal von anderen Signalen unterscheiden zu können.

Bei den oben dargestellten Ausführungsbeispielen hatte der Pegelstand der Blasenkammer dabei jeweils einen Einfluss auf zumindest einen Teil des von dem Körperschallemitter 24 ausgesendeten und am Körperschallsensor 26 ankommenden Signals.

Wird jedoch bei dem in Figur 1 gezeigten Ausführungsbeispiel als Blutpumpe 14 eine okkludierende Pumpe eingesetzt, blockiert diese das in Flussrichtung durch den extrakorporalen Blutschlauchsatz sich ausbreitende Signal des Körperschallemitters 24 weitgehend, so dass dieses beinahe ausschließlich über den Patientenzugang 30 zum Körperschallsensor 26 gelangt. Die Blutpumpe 14 erzeugt jedoch ebenfalls Druckpulse, welche sich in beide Richtungen durch den Kreislauf ausbreiten. Auf einem ersten Signalweg durchlaufen die Druckpulsationen, welche ebenfalls Körperschall im Sinne der vorliegenden Erfindung darstellen, den Dialysator 16 und gelangen über die Blasenkammer 20 zum Körperschallsensor 26. Auf dem zweiten Signalweg breiten sich die Druckpulsationen der Pumpe über den Patientenzugang 30 und die venöse Leitung 22 zum Körperschallsensor 26 aus.

Bevorzugt wird der Körperschallemitter 24 daher so betrieben, dass dessen Signal deutlich von dem Signal der Blutpumpe unterscheidbar ist. Insbesondere kann hier eine andere Frequenz eingesetzt werden als jene, welche die Blutpumpe erzeugt. Die beiden auf den Körperschallemitter 24 und die Blutpumpe beruhenden Signalanteile können daher aus dem vom Körperschallsensor 26 gemessenen Signal separiert werden und separat ausgewertet werden, beispielsweise durch eine entsprechende Frequenzseparierung. Das vom Körperschallemitter 24 erzeugte Signal kann dann ausschließlich zur Detektion einer Nadeldiskonnektion eingesetzt werden. Das Signal der als zweiter Körperschallemitter dienenden Blutpumpe 14 kann dann zur Bestimmung des Pegelstandes, und gegebenenfalls zusätzlich zu einer zweiten Detektionsstufe für die Diskonnektion des Patienten eingesetzt werden.

Unabhängig von dem konkreten Aufbau können daher die Vorgänge, welche das Signal am Körperschallsensor 26 verändern, entweder einer Pegelstandsänderung in der Tropfkammer oder einer Nadeldiskonnektion zugeordnet werden. Das jeweils andere Signal kann dabei auch als Referenz für eine Seite dienen, z. B. zur Bestimmung einer Baseline. Es kann auch eine gemeinsame Auswertung einer Funktion erfolgen, die von beiden Signalen abhängt, z. B. als Quotient.

## Patentansprüche

1. Medizinisches Gerät, insbesondere Blutbehandlungsvorrichtung, mit einer Steuer- und Recheneinheit, mindestens einem Körperschallemitter (24) und mindestens einem Körperschallsensor (26) jeweils konfiguriert zum Ankoppeln an Ankoppelstellen eines an das medizinische Gerät ankoppelbaren medizinischen Schlauchsatzes,
**dadurch gekennzeichnet,**
**dass** über die Steuer-und Recheneinheit der Füllstand einer im Schlauchsatz angeordneten Blasenkammer (20) auf der Grundlage der Messung des Körperschalls am Schlauchsatz bestimmbar ist, indem über den Körperschallemitter ein Körperschall über eine Ankoppelstelle in den Schlauchsatz vor der Blasenkammer eingekoppelt wird und der sich im Fluidkreislauf ausbreitende Körperschall über den an eine in Körperschallausbreitungsrichtung nach der Blasenkammer angeordnete Ankoppelstelle gekoppelten Körperschallsensor aufgenommen und der Steuer- und Recheneinheit zur Bestimmung der Füllhöhe zugeleitet wird.

2. Medizinisches Gerät nach Anspruch 1, **dadurch gekennzeichnet, dass** die Steuer-und Recheneinheit die Füllhöhe der Blasenkammer durch Vergleich mit mindestens einer abgespeicherten Kennlinie bestimmt.

3. Medizinisches Gerät nach einem der vorangegangenen Ansprüche, wobei es sich bei dem Körperschallemitter um eine okkludierende Pumpe, insbesondere eine Rollenpumpe handelt, welche an einen Pumpabschnitt des Schlauchsatzes ankoppelbar ist, und/oder wobei es sich bei dem Körperschallemitter um einen elektronisch angesteuerten Schallerzeuger handelt, welcher bevorzugt ein Piezoelement umfasst und/oder bevorzugt stromaufwärts oder stromabwärts einer Pumpe am Schlauchsatz ankoppelbar ist.

4. Medizinisches Gerät nach einem der vorangegangenen Ansprüche, wobei es sich bei dem medizinischen Gerät um eine Blutbehandlungsvorrichtung für die extrakorporale Blutbehandlung und bei dem Schlauchsatz um einen extrakorporalen Blutschlauchsatz handelt.

5. Medizinisches Gerät nach Anspruch 4, **dadurch gekennzeichnet, dass** die Steuer-und Recheneinheit weiterhin den Zustand eines Patientenzugangs (30) überwacht, wobei die Steuer- und Recheneinheit bevorzugt eine Nadeldiskonnektion detektiert, wenn sich die Schallausbreitung im extrakorporalen Blutkreislauf aufgrund des fehlerhaften Patientenzugangs ändert und/oder die gemessene Amplitude des Körperschalls abnimmt oder ausbleibt, wobei sie bevorzugt bei Detektion einer Nadeldiskonnektion die Blutpumpe stoppt und/oder einen Alarm auslöst.

6. Medizinisches Gerät nach Anspruch 5, wobei das Signal des Körperschallsensors zur Überwachung des Patientenzugangs und zur gleichzeitigen Bestimmung der Füllhöhe der Blasenkammer in unterschiedlicher Weise ausgewertet wird, wobei bevorzugt unterschiedliche Signalanteile und/oder sich in ihrem zeitlichen Verlauf unterscheidende Änderungen des Signals den beiden unterschiedlichen Aspekten zugeordnet werden.

7. Medizinisches Gerät nach Anspruch 6, wobei das Signal zur Detektion einer Nadeldiskonnektion auf schnellere Amplitudenänderungen hin überwacht wird, wofür das aktuelle gemessene Signal bevorzugt mit einem Basissignal verglichen wird, welches bevorzugt während des Behandlungsverlaufs aktualisiert wird und/oder auf Grundlage eines zeitlichen Mittelwertes gebildet wird, während Änderungen der Füllhöhe der Blasenkammer anhand langsamerer Amplitudenänderungen erfasst werden, insbesondere anhand eines Basissignals, welches auf Grundlage eines zeitlichen Mittelwertes gebildet wird.

8. Medizinisches Gerät nach Anspruch 6, wobei zwei Körperschallemitter mit unterschiedlichen Signalverläufen eingesetzt werden, insbesondere eine okkludierende Blutpumpe und ein stromaufwärts der Blutpumpe angeordneter weiterer Körperschallemitter, wobei die jeweils auf die Körperschallemitter entfallenden Signalanteile getrennt werden und zur Überwachung des Patientenzugangs sowie zur gleichzeitigen der Bestimmung der Füllhöhe der Blasenkammer separat ausgewertet werden.

9. Medizinisches Gerät nach einem der vorangegangenen Ansprüche, **dadurch gekennzeichnet, dass** der mindestens eine Emitter und der mindestens eine Sensor in das medizinische Gerät derart integriert sind, dass diese beim Einlegen des Schlauchsatzes jeweils an vorgesehenen Ankoppelstellen an den Schlauchsatz ankoppelbar sind.

10. Medizinisches Gerät nach einem der vorangehenden Ansprüche mit einem Schlauchsatz, **dadurch gekennzeichnet, dass** der Schlauchsatz mindestens eine Ankoppelstelle für einen Körperschallemitter, mindestens eine Blasenkammer und mindestens eine Ankoppelstelle für einen Körperschallsensor aufweist.

11. Medizinisches Gerät nach Anspruch 10, **dadurch gekennzeichnet, dass** die Ankoppelstelle für den Körperschallemitter am arteriellen Zweig des Blutschlauchsatzes stromaufwärts der Blutpumpe angeordnet ist und/oder **dadurch gekennzeichnet, dass** ein Pumpsegment des Blutschlauchsatzes als Ankoppelstelle für die als Körperschallemitter dienende Blutpumpe eingesetzt wird, und/oder **dadurch gekennzeichnet, dass** die Ankoppelstelle für den Körperschallsensor stromabwärts oder stromaufwärts der venösen Blasenkammer angeordnet ist.

12. Verfahren zur Detektion des Füllstandes einer Blasenkammer in einem Fluidkreislauf eines medizinischen Gerätes, insbesondere einem extrakorporalen Blutkreislauf in einer Blutbehandlungsvorrichtung, **dadurch gekennzeichnet, dass** über einen Körperschallemitter ein Körperschall über eine Ankoppelstelle in einen Schlauchsatz, insbesondere einen extrakorporalen Blutschlauchsatz, vor der Blasenkammer eingekoppelt wird und dass der sich im Fluidkreislauf ausbreitende Körperschall über einen an eine in Körperschallausbreitungsrichtung nach der Blasenkammer angeordnete Ankoppelstelle gekoppelten Körperschallsensor aufgenommen und einer Steuer- und Recheneinheit zur Bestimmung der Füllhöhe zugeleitet wird.

13. Verfahren nach Anspruch 12, **dadurch gekennzeichnet, dass** in einer Steuer-und Recheneinheit der Blutbehandlungsvorrichtung die Füllhöhe der Flüssigkeit in der Blasenkammer durch Vergleich mit abgespeicherten Kennlinien bestimmt wird.

14. Verfahren nach Anspruch 12 oder 13 zur Detektion des Füllstandes einer Blasenkammer in einem extrakorporalen Blutkreislauf in einer Blutbehandlungsvorrichtung nach einem der vorangegangenen Ansprüche.

15. Computerprogrammprodukt mit einem Quellcode zur Ausführung des Verfahrens nach einem der vorangehenden Ansprüche, wenn das Computerprogramm in einer Steuer- und Recheneinheit eines medizinischen Gerätes abläuft.

## Claims

1. Medical device, in particular blood treatment apparatus, having a control and processing unit, at least one structure-borne sound emitter (24) and at least one structure-borne sound sensor (26), each configured for coupling to coupling points of a medical tubing kit which can be coupled to the medical device, **characterized in that**
the filling status of a bubble chamber (20) arranged in the tubing kit can be determined via the control and processing unit on the basis of the measurement of the structure-borne sound at the tubing kit by coupling, via the structure-borne sound emitter, a structure-borne sound into the tubing kit upstream the bubble chamber via a coupling point and the structure-borne sound which propagates in the fluid circuit is taken up via the structure-borne sound sensor coupled to a coupling point arranged downstream the bubble chamber in structure-borne sound propagation direction and is supplied to the control and processing unit for determining the filling level.

2. Medical device according to claim 1, **characterized in that** the control and processing unit determines the filling level of the bubble chamber by comparing with at least one stored characteristic.

3. Medical device according to one of the preceding claims, wherein the structure-borne sound emitter is an occluding pump, in particular a roller pump, which can be coupled to a pump section of the tubing kit, and/or wherein the structure-borne sound emitter is an electronically controlled sound generator which preferably comprises a piezo-element and/or which can preferably be coupled upstream or downstream of a pump at the tubing kit.

4. Medical device according to one of the preceding claims, wherein the medical device is a blood treatment apparatus for the extracorporeal blood treatment and the tubing kit is an extracorporeal blood tubing kit.

5. Medical device according to claim 4, **characterized in that** the control and processing unit furthermore monitors the status of a patient port (30), wherein the control and processing unit preferably detects a needle disconnection when the sound propagation in the extracorporeal blood circuit changes due to the defective patient port and/or when the measured amplitude of the structure-borne sound reduces or is missing, wherein it preferably stops the blood pump and/or triggers an alarm on detection of a needle disconnection.

6. Medical device according to claim 5, wherein the signal of the structure-borne sound sensor is evaluated in different manners for monitoring the patient port and for the simultaneous determination of the filling level of the bubble chamber, wherein different signal portions and/or changes in the signal differing in their time development are preferably associated with the two different aspects.

7. Medical device according to claim 6, wherein the signal is monitored for faster amplitude changes for detection of a needle disconnection, for which purpose the currently measured signal is preferably compared with a base signal which is preferably updated during the course of the treatment and/or is formed on the basis of an average time value, whereas changes in the filling level of the bubble chamber are detected with reference to slower amplitude changes, in particular with reference to a base signal which is formed on the basis of an average time value.

8. Medical device according to claim 6, wherein two structure-borne sound emitters having different signal developments are used, in particular an occluding blood pump and a further structure-borne sound emitter arranged upstream of the blood pump, wherein the respective signal portions attributable to the structure-borne sound emitters are separated and are evaluated separately for monitoring the patient port and for the simultaneous determination of the filling level of the bubble chamber.

9. Medical device according to one of the preceding claims, **characterized in that** the at least one emitter and the at least one sensor are integrated in the medical device such that they can be coupled to the tubing kit at respective provided coupling points upon insertion of the tubing kit.

10. Medical device according to one of the preceding claims having a tubing kit, **characterized in that** the tubing kit comprises at least one coupling point for a structure-borne sound emitter, at least one bubble chamber and at least one coupling point for a structure-borne sound sensor.

11. Medical device according to claim 10, **characterized in that** the coupling point for the structure-borne sound emitter at the arterial branch of the blood tubing kit is arranged upstream of the blood pump and/or is **characterized in that** a pump segment of the blood tubing kit is used as a coupling point for the blood pump serving as the structure-borne sound emitter, and/or **characterized in that** the coupling point for the structure-borne sound sensor is arranged downstream or upstream of the venous bubble chamber.

12. Method for detecting the filling level of a bubble chamber in a fluid circuit of a medical device, in particular in an extracorporeal blood circuit in a blood treatment apparatus, **characterized in that** a structure-borne sound is coupled into a tubing kit by a structure-borne sound emitter via a coupling point in particular into an extracorporeal blood tubing kit, before the bubble chamber and **in that** the structure-borne sound propagating in the fluid circuit is taken up by a structure-borne sound sensor coupled to a coupling point arranged in the structure-borne sound propagation direction after the bubble chamber and is conducted to a control and processing unit for determining the filling level.

13. Method according to claim 12, **characterized in that** in a control and processing unit of the blood treatment apparatus the filling level of the liquid in the bubble chamber is determined by comparing with stored characteristics.

14. Method according to claim 12 or 13 for detecting the filling level of a bubble chamber in an extracorporeal blood circuit in a blood treatment apparatus according to one of the preceding claims.

15. Computer program product comprising a source code for carrying out the method according to one of the preceding claims when the computer program runs in a control and processing unit of a medical device.

## Revendications

1. Appareil médical, notamment dispositif de traitement de sang, comprenant une unité de commande et de calcul, au moins un émetteur de bruit de structure (24) et au moins un capteur de bruit de structure (26), respectivement configurés pour le couplage à des points de couplage d'un jeu de tuyaux médicaux pouvant être couplés à l'appareil médical,
**caractérisé en ce**
**que** le niveau de remplissage d'une chambre à bulles (20) disposée dans le jeu de tuyaux peut être déterminé sur le jeu de tuyaux, sur la base de la mesure du bruit de structure, par l'intermédiaire de l'unité de commande et de calcul du fait que, par l'intermédiaire de l'émetteur de bruit de structure, un bruit de structure est couplé en amont de la chambre à bulles par le biais d'un point de couplage dans le jeu de tuyaux et que le bruit de structure se propageant dans le circuit de fluide est enregistré par l'intermédiaire du capteur de bruit de structure couplé à un point de couplage disposé en aval de la chambre à bulles en direction de propagation du bruit de structure et est amené à l'unité de commande et de calcul pour la détermination de la hauteur de remplissage.

2. Appareil médical selon la revendication 1, **caractérisé en ce que** l'unité de commande et de calcul détermine la hauteur de remplissage de la chambre à bulles par comparaison avec au moins une courbe caractéristique mémorisée.

3. Appareil médical selon l'une quelconque des revendications précédentes, l'émetteur de bruit de structure étant une pompe à occlusion, notamment une pompe à rouleaux, laquelle peut être couplée à une section de pompage du jeu de tuyaux, et/ou l'émetteur de bruit de structure étant un générateur sonore à commande électronique, lequel comprend de préférence un élément piézoélectrique et/ou peut être couplé au jeu de tuyaux, de préférence en amont ou en aval d'une pompe.

4. Appareil médical selon l'une quelconque des revendications précédentes, l'appareil médical étant un dispositif de traitement de sang pour le traitement extracorporel du sang, et le jeu de tuyaux étant un jeu de tuyaux extracorporel pour sang.

5. Appareil médical selon la revendication 4, **caractérisé en ce que** l'unité de commande et de calcul surveille en outre l'état d'un accès (30) sur le patient, l'unité de commande et de calcul détectant de préférence une déconnexion d'aiguille lorsque la propagation du son dans la circulation extracorporelle du sang se modifie en raison de la défectuosité de l'accès sur le patient et/ou lorsque l'amplitude mesurée du bruit de structure diminue ou est absente, l'unité de commande et de calcul, de préférence lors de la détection d'une déconnexion d'aiguille, arrêtant la pompe à sang et/ou déclenchant une alarme.

6. Appareil médical selon la revendication 5, le signal du capteur de bruit de structure destiné à surveiller l'accès sur le patient et à déterminer simultanément la hauteur de remplissage de la chambre à bulles étant évalué de manière différente, de préférence des fractions de signal différentes et/ou des modifications du signal se différenciant dans leur allure temporelle étant affectées aux deux aspects différents.

7. Appareil médical selon la revendication 6, le signal de détection d'une déconnexion d'aiguille étant surveillé quant à des modifications d'amplitude plus rapides, sur quoi le signal mesuré momentanément est de préférence comparé à un signal de base, lequel est mis à jour de préférence pendant le déroulement du traitement et/ou est formé sur la base d'une valeur temporelle moyenne pendant des modifications de la hauteur de remplissage de la chambre à bulles à l'aide de modifications d'amplitudes plus lentes, notamment à l'aide d'un signal de base, lequel est formé sur la base d'une valeur temporelle moyenne.

8. Appareil médical selon la revendication 6, deux émetteurs de bruit de structure ayant différentes courbes de signal étant utilisés, notamment une pompe à sang à occlusion et un émetteur de bruit de structure supplémentaire disposé en amont de la pompe à sang, les fractions de signal revenant respectivement à l'émetteur de bruit de structure étant séparées et évaluées à part pour la surveillance de l'accès sur le patient ainsi que pour la détermination simultanée de la hauteur de remplissage de la chambre à bulles.

9. Appareil médical selon l'une quelconque des revendications précédentes, **caractérisé en ce que** l'au moins un émetteur et l'au moins un capteur sont intégrés dans l'appareil médical de manière à ce que ceux-ci, lors de la pose du jeu de tuyaux, puissent être respectivement couplés au jeu de tuyaux à des points de couplage ménagés.

10. Appareil médical selon l'une quelconque des revendications précédentes comprenant un jeu de tuyaux, **caractérisé en ce que** le jeu de tuyaux présente au moins un point de couplage pour un émetteur de bruit de structure, au moins une chambre à bulles et au moins un point de couplage pour un capteur de bruit de structure.

11. Appareil médical selon la revendication 10, **caractérisé en ce que** le point de couplage pour l'émetteur de bruit de structure est disposé en amont de la pompe à sang, sur l'embranchement artériel du jeu de tuyaux pour sang, et/ou **caractérisé en ce qu'**un segment de pompage du jeu de tuyaux pour sang est utilisé comme point de couplage pour la pompe à sang servant d'émetteur de bruit de structure, et/ou **caractérisé en ce que** le point de couplage pour le capteur de bruit de structure est disposé en amont ou en aval de la chambre à bulles veineuse.

12. Procédé de détection du niveau de remplissage d'une chambre à bulles dans un circuit de fluide d'un appareil médical, notamment dans un circuit de sang extracorporel dans un dispositif de traitement de sang, **caractérisé en ce que**, par l'intermédiaire d'un émetteur de bruit de structure, un bruit de structure est couplé en amont de la chambre à bulles par le biais d'un point de couplage dans un jeu de tuyaux, notamment dans un jeu de tuyaux extracorporel pour sang, et **en ce que** le bruit de structure se propageant dans le circuit de fluide est enregistré par l'intermédiaire d'un capteur de bruit de structure couplé à un point de couplage disposé en aval de la chambre à bulles en direction de propagation du bruit de structure et est amené à une unité de commande et de calcul pour la détermination de la hauteur de remplissage.

13. Procédé selon la revendication 12, **caractérisé en ce que** dans une unité de commande et de calcul du dispositif de traitement de sang, la hauteur de remplissage du liquide dans la chambre à bulles est déterminée par comparaison avec des courbes caractéristiques mémorisées.

14. Procédé selon la revendication 12 ou 13, destiné à la détection du niveau de remplissage d'une chambre à bulles dans un circuit de sang extracorporel dans un dispositif de traitement de sang selon l'une quelconque des revendications précédentes.

15. Produit informatique comprenant un code source destiné à la réalisation du procédé selon l'une quelconque des revendications précédentes, lorsque le produit informatique se déroule dans une unité de commande et de calcul d'un appareil médical.
